# EUROPÄISCHE PATENTSCHRIFT

(11) **EP 1 572 354 B1**
(45) Veröffentlichungstag und Bekanntmachung des Hinweises auf die Patenterteilung: **07.06.2006**
(21) Anmeldenummer: 03810970.8
(22) Anmeldetag: 06.11.2003
(51) Int. Cl.: B01J 23/656, B01J 23/68, C07D 307/08, C07D 315/00

(54) **RHENIUMHALTIGER TRÄGERKATALYSATOR UND VERFAHREN ZUR HYDRIERUNG VON CARBONYLVERBINDUNGEN IN FLÜSSIGER PHASE UNTER VERWENDUNG DES KATALYSATORS**
SUPPORTED CATALYST CONTAINING RHENIUM AND METHOD FOR HYDROGENATION OF CARBONYL COMPOUNDS IN LIQUID PHASE BY MEANS OF SAID CATALYST
CATALYSEUR SUPPORTE CONTENANT DU RHENIUM ET PROCEDE D'HYDROGENATION DE COMPOSES CARBONYLES EN PHASE LIQUIDE AU MOYEN DUDIT CATALYSEUR

(30) Priorität: 11.11.2002 DE 10252281
(43) Veröffentlichungstag der Anmeldung: 14.09.2005
(73) Patentinhaber: BASF Aktiengesellschaft, 67056 Ludwigshafen (DE)
(72) Erfinder: SCHUBERT, Markus, 67063 Ludwigshafen (DE); HESSE, Michael, 67549 Worms (DE); FISCHER, Rolf-Hartmuth, 69121 Heidelberg (DE); CONSTANTINESCU, Take, 69121 Heidelberg (DE)
(86) Internationale Anmeldenummer: PCT/EP2003/012379
(87) Internationale Veröffentlichungsnummer: WO 2004/043592

(56) Entgegenhaltungen:
- EP-A- 1 112 776
- US-A- 3 840 475
- PRESTVIK R ET AL: "Bimetallic Particle Formation in Pt-Re/Al2O3Reforming Catalysts Revealed by Energy-Dispersive X-Ray Spectrometry in the Analytical Electron Microscope" JOURNAL OF CATALYSIS, ACADEMIC PRESS, DULUTH, MN, US, Bd. 176, Nr. 1, 15. Mai 1998 (1998-05-15), Seiten 246-252, XP004447412 ISSN: 0021-9517

## Beschreibung

Die Erfindung betrifft ein Verfahren zur Hydrierung von Carbonylgruppen enthaltenden Verbindungen in der Flüssigphase an rheniumhaltigen Trägerkatalysatoren, die mindestens ein weiteres Aktivmetall aufweisen, das zusammen mit dem Rhenium in Form einer bimetallischen Verbindung auf den Träger aufgebracht wird.

Die industrielle Hydrierung von Carbonylgruppen enthaltenden Verbindungen wie Aldehyden, Ketonen, Carbonsäuren, Carbonsäureanhydriden mit Wasserstoff an rheniumhaltigen Katalysatoren ist seit langem bekannt.

So beschreibt DE-A 100 09 817 einen rheniumhaltigen Trägerkatalysator, bei dem als Trägermaterial nichtoxidativ vorgehandelte Aktivkohle verwendet wird. Die Katalysatoren enthalten zudem weitere Übergangsmetalle, insbesondere Platinmetalle, um die Katalysatoraktivität zu steigern. Rhenium und die weiteren Übergangsmetalle werden dabei auf den Träger in Form getrennter oder gemeinsamer Lösungen ihrer jeweiligen Salze aufgebracht. Die beschriebenen Hydrierungen ergeben als Hauptprodukt Alkohole.

Aus DE-A 2 519 817 sind Katalysatoren bekannt, die gleichzeitig Elemente der Gruppe VII und VIII des Periodensystems der Elemente enthalten. Bevorzugt sind rheniumhaltige, zusätzlich Platin oder Palladium aufweisende Trägerkatalysatoren. Diese Katalysatoren weisen insbesondere Rhenium und Palladium auf, die im Verlauf der Katalysatorherstellung gemäß den Ausführungsbeispielen bevorzugt, gleichzeitig auf den Träger aufgebracht werden. Es ist gemäß der Offenbarung der DE-A 2 519 817 auch möglich die Palladiumverbindung zuerst auf den Träger aufzubringen. Die Aktivität der geträgerten Palladium-Rhenium-Katalysatoren in Hydrierungen von Carbonylgruppen enthaltender Verbindung zu Alkoholen ist so gering, dass die gleichzeitige Anwendung von hohen Drücken und hohen Temperaturen von 215 bis 230°C erforderlich wird. Die Durchführung der Hydrierungen bei hohen Drücken und hohen Temperaturen ist bedingt durch hohe Energie- und Materialkosten wenig wirtschaftlich. Zudem nimmt die Korrosität insbesondere der Carbonsäure-Lösungen unter diesen Bedingungen zu.

Aus EP-A 1 112 776 ist ein Verfahren zur Hydrierung von C₄-Dicarbonsäuren, deren Anhydriden oder Estern unter Verwendung eines Katalysators bekannt, bei dem die Rheniumkomponente sehr gleichmäßig auf dem Trägermaterial verteilt ist. Die zusätzlich vorhandene Palladium-komponente zeigt jedoch ein deutliches Schalenprofil, so dass der Synergieeffekt, der vermutlich auf Bildung einer intermetallischen Phase besteht, nur begrenzt ausgenutzt wird. Mit den beschriebenen Katalysatoren wird gamma-Butyrolacton mit guter Selektivität gebildet. Die Produktgemische enthalten jedoch nur Spuren von THF.

Es ist die Aufgabe der vorliegenden Erfindung einen Katalysator für die Hydrierung von Carbonylverbindungen sowie ein Verfahren zur Hydrierung von Carbonylverbindungen, insbesondere Dicarbonsäuren wie Maleinsäure und/oder Bernsteinsäure oder deren Anhydriden oder Estern unter Verwendung dieses Katalysators zur Verfügung zu stellen, mit dem insbesondere Gemische mit etwa gleichen Anteilen von gegebenenfalls substituiertes gamma-Butyrolacton (in folgenden "GBL") und Tetrahydrofuran (im folgenden "THF") herstellbar sind und dass es gestattet diese Gemische bei gutem Umsatz mit guter Gesamtselektivität herzustellen.

Die Aufgabe wird gelöst durch einen rheniumhaltigen Trägerkatalysator zur Hydrierung von Carbonylverbindungen wie Dicarbonsäuren und/oder deren Derivaten, insbesondere von Maleinsäure und/oder Bersteinsäure, deren Anhydriden und/oder Estern insbesondere zu Gemischen von gegebenenfalls substituierten Y-Butyrolacton und Tetrahydrofuran, der dadurch gekennzeichnet ist, dass Rhenium und mindestens ein weiteres Metall der Gruppen VIII oder Ib des Periodensystems der Elemente, insbesondere Ruthenium (Ru), Rhodium (Rh), Palladium (Pd), Osmium (Os), Iridium (Ir), Platin (Pt), Kupfer (Cu), Silber (Ag) oder Cobalt (Co) in Form mindestens einer bimetallischen Vorläuferverbindung auf den Träger aufgebracht wurde, wobei als bimetallische Vorläuferverbindung Perrhenat-Doppelsalze, der allgemeinen Formel I

[Meₐ (NH₃)_{b} (OH)_{c}] (ReO₄)_{d} • eH₂O (I)

oder deren Gemische eingesetzt werden, wobei Me für ein Metall der Gruppen VIII und Ib des Periodensystems der Elemente, insbesondere für Ru (Ruthenium), Rh (Rhodium), Pd (Palladium), Os (Osmium), Ir (Iridium), Pt (Platin), Cu (Kupfer), Ni (Nickel), Ag (Silber) oder Co (Cobalt) steht, a 1 oder 2 bedeutet, b eine ganze Zahl von 1 bis 8, c eine ganze Zahl von 0 bis 5, d 2, 3 oder 4 und e eine ganze Zahl von 0 bis 12 darstellt.

Doppelsalze sind Mischkristalle zweier Salze. Anionen- und Kationen eines lonenkristalls können durch andere Kationen und Anionen ersetzt werden, ohne dass sich der Kristallstrukturtyp ändert. Ist das sich gegenseitig vertretende lonenpaar nicht rein statisch, sondern nach einem bestimmten Verteilungsplan im lonengitter angeordnet, resultiert ein Doppelsalz. Die Herstellung solcher Doppelsalze ist an sich bekannt und beispielsweise von Pechenyuk, S.I., Kuznetsov, V.Y., Popova, R.A., Zalkind, O.A., Zh. Neorg. Khim. 24 (1979) 3306 beschrieben.

Erfindungsgemäß wurde erkannt, dass durch Einsatz dieser Doppelsalze zur Aufbringung der katalytisch aktiven Komponenten auf den Träger eine gleichförmige Verteilung aller katalytisch aktiven Metalle erreicht wird.

Besonders bevorzugt wird als bimetallische Vorläuferverbindung Pd (NH₃)₄ (ReO₄)₂ und/oder Pt (NH₃)₄ (ReO₄)₂ verwendet.

Als Trägermaterial kommen alle für die Herstellung von Hydrierkatalysatoren bekannten Trägermaterialien in Frage. Bevorzugt werden Siliziumoxid, Aluminiumoxid, Titandioxid, Zikondioxid, Magnesiumoxid, gegebenenfalls vorbehandelter Aktivkohle, graphitischen Kohleträgern, Nitride, Silicid, Carbide oder Boride. Bei der erwähnten Vorbehandlung kann es sich um eine oxidative Vorbehandlung, wie sie beispielsweise in EP-A 848 991 beschrieben ist, handeln. Bevorzugt werden Träger aus gegebenenfalls vorbehandelter Aktivkohle eingesetzt.

Rhenium (Re, als Metall gerechnet) und das weitere Metall der Gruppe VIII oder Ib des Periodensystems sind in einer Menge von jeweils 0,03 bis 30 Gew.-%, bevorzugt 1 bis 12 Gew.-%, besonders bevorzugt 2 bis 5 Gew.-%, bezogen auf den gesamten Katalysator aus Träger und Aktivmasse, aufgebracht.

Es können auf dem Katalysator noch weitere Elemente vorhanden sein. Beispielhaft seien Zn (Zink), Sn (Zinn), Au (Gold), Fe (Eisen), Mn (Mangan), Cr (Chrom), Mo (Molybdan), W (Wolfram) und V (Vanadium) genannt. Ebenso können noch Elemente der Gruppen VII, VIII oder Ib des Periodensystems der Elemente wie Rhenium (Re), Platin (Pt), Ruthenium (Ru), Silber (Ag) und Palladium (Pd) zusätzlich vorhanden sein. Diese Elemente modifizieren den Katalysator im wesentlichen bzgl. Aktivität und Selektivität (Hydrogenolyseprodukte) sind aber nicht essentiell. Ihr Gewichtsverhältnis zu Rhenium kann 0 bis 100, bevorzugt 0,5 bis 30, besonders bevorzugt 0,1 bis 5 betragen. Bevorzugt sind die erfindungsgemäßen Katalysatoren insbesondere chromfrei.

Die Aufbringung der Aktivkomponenten Rhenium und des weiteren Metalls der Gruppe VIII oder Ib des Periodensystems der Elemente kann durch Imprägnierung in einem oder mehreren Schritten mit einer wässrigen, alkoholischen oder mit anderen organischen Lösungsmittein hergestellten Lösung der jeweiligen gelösten bimetallischen Vorläuferverbindung, in Form des Doppelsalzes der allgemeinen Formel I, Gleichgewichtsadsorption in einem oder mehreren Schritten der in wässriger oder alkoholischer Lösung gelösten bimetallischen Vorläuferverbindung in Form des Doppelsalzes der allgemeinen Formel I an den Träger vorgenommen werden. Bei diesen Verfahren werden die Aktivkomponenten gleichzeitig und gleichmäßig auf das Trägermaterial aufgebracht werden. Zwischen den einzelnen Imprägnierungs- und Gleichgewichtsadsorptionsschritten liegt jeweils ein Trocknungsschritt zur Entfernung des Lösungsmittels. Bevorzugt geschieht die Aufbringung der Aktivkomponenten durch Imprägnierung mit einer wässrigen Salzlösung in einem Schritt.

Zur Entfernung des Lösungsmittels nach dem Imprägnierungs- oder Gleichgewichtsadsorptionsschritt erfolgt eine Trocknung des imprägnierten Katalysators. Die Trocknungs-temperatur liegt dabei bei 30 - 350°C, bevorzugt 40 - 280°C, besonders bevorzugt 50 - 150°C.

Die Aktivkomponenten sind auf dem Träger des erfindungsgemäßen Katalysators besonders gleichmäßig verteilt, insbesondere weist das Intensitäsverhältnis von Rhenium zum Metall (Me) der Gruppe VIII oder Ib des Periodensystems der Elemente über die gesamten Katalysatorpartikel bei mehr als 99,9 % der analysierten Punkte Schwankungen kleiner als den Faktor 10, bezogen auf den statistischen Mittelwert, bevorzugt bei 98 % der analysierten Punkte auf der Katalysatoroberfläche Schwankungen kleiner als Faktor 5 und besonders bevorzugt in 80 % der analysierten Punkte Schwankungen kleiner als Faktor 2, auf.

Dieser Faktor wurde mit SEM-EDX (Scanning electron microscope-energy dispersive x-ray spectroscopy) bestimmt. Die Methode ist an sich bekannt und beispielsweise in Ulmanns Encylopedia of Industrial Chemistry 6^{th} Edition 2000 Electronic Release beschrieben.

Die Katalysatoren werden üblicherweise vor ihrem Einsatz aktiviert. Diese Aktivierung kann durch Anwendung einer reduzierend wirkenden Gasatmosphäre auf den Katalysator geschehen. Bevorzugt wird eine Aktivierung mit Hilfe von Wasserstoff angewendet. Die Aktivierungs-temperatur liegt dabei üblicherweise bei 100 - 500°C, bevorzugt 130 - 400°C, besonders bevorzugt 150 - 400°C. Alternative Reduktionsmethoden sind die Reduktion der metallischen Komponenten durch in Kontaktbringen mit einem flüssigen Reduktionsmittel wie Hydrazin, Formaldehyd oder Natriumformiat. Dabei werden die flüssigen Reduktionsmittel üblicherweise bei Temperaturen zwischen 10 und 100°C in Kontakt gebracht. Besonders bevorzugt ist das Inkontaktbringen bei Temperaturen zwischen 20 bis 80°C.

Die Hydrierung wird üblicherweise bei 110 - 250°C, bevorzugt bei 150 - 250°C durchgeführt. Dabei wird üblicherweise bei einem Reaktionsdruck zwischen 5 und 220 bar, bevorzugt 40 und 150 bar hydriert. Die Hydrierung wird in der Flüssigphase bevorzugt im Festbett durchgeführt.

Als Ausgangsstoffe für die Hydrierung sind im allgemeinen Carbonylverbindungen geeignet, die zusätzlich C-C-Doppel- oder Dreifachbindungen enthalten können. Beispiel für Aldehyde sind Propionaldehyd, Butyraldehyde, Crotonaldehyd, Ethylhexanal, Nonanal und Glucose. Beispiele für Carbonsäuren sind Bernsteinsäure, Fumarsäure, Maleinsäure. Als Ester sind Ester der vorgenannten Säuren, z.B. als Methyl-, Ethyl-, Propyl- oder Butylester zu nennen, ferner sind Lactone, z.B. gamma-Butyrolacton, delta-Valerolacton oder Caprolacton einsetzbar. Außerdem können Anhydride wie Bernsteinsäureanhydrid oder Maleinsäureanhydrid verwendet werden. Bevorzugte Ausgangsstoffe sind C₄-Dicarbonsäuren und/oder deren Derivate, besonders bevorzugt Bernsteinsäure, Maleinsäure, Bernsteinsäureanhydrid, Maleinsäureanhydrid sowie die Ester dieser Säuren. Es können selbstverständlich auch Gemische von Aldehyden, Carbonsäuren, Estern, Anhydriden und/oder Lactonen, bevorzugt Gemische von Carbonsäuren, eingesetzt werden.

Die zu hydrierenden Verbindungen können in Substanz oder in Lösung hydriert werden. Als Lösungsmittel bietet sich z.B. eines der Hydrierprodukte selbst an, oder es werden Stoffe eingesetzt wie Alkohole wie Methanol, Ethanol, Propanol oder Butanol, ferner sind Ether wie THF oder Ethylenglycolether oder gamma-Butyrolacton geeignet. Ein bevorzugtes Lösungsmittel ist Wasser, insbesondere bei der Hydrierung von Carbonsäuren.

Die Hydrierung kann in der Flüssigphase, ein- oder mehrstufig ausgeübt werden. In der Flüssigphase ist sowohl die Suspensions- als auch die Festbettfahrweise möglich. Bei exothermen Reaktionen kann die Wärme durch außenliegende Kühlmittel abgeführt werden (z.B. Röhrenreaktor). Ferner ist Siedekühlung im Reaktor möglich, vor allem wenn ohne Produktrückführung hydriert werden. Bei Produktrückführung bietet sich ein Kühler im Rückführstrom an.

Das erfindungsgemäße Verfahren wird anhand der nachstehenden Beispiele erläutert.

### Beispiele

### Bestimmung des Intensitätsfaktors mit SEM-EDX

Mit einem SEM-EDX Spektrometer des Typs Philips ESEM-XL30-FG mit EDX-Sonde wurden die Intensitäten (entsprechend den Gehalten) des Rheniums und des Palladiums bestimmt. Die Analysenspannung betrug 30 KV. Die Partikel wurden zur Vorbereitung so geteilt, dass eine saubere Schnittfläche erhalten wurde. Von dieser Schnittfläche wurden mehr als 300 µm in Schritten zu je 15 µm mit SEM-EDX auf ihren Gehalt an Pd und Re untersucht. An jedem Messpunkt kann ein Verhältnis der Intensitäten von Pd und Re berechnet werden.

### Beispiel 1

15,83 g Pd(NO₃)₂ wurden mit 8 g 25%iger NH₃-Lösung versetzt und mit einer Lösung von 8,66 g NH₄ReO₄ in 98 g Wasser vermischt. Die Verbindung Pd(NO₃)₂ (ReO₄)₂ kristallisierte aus. Das durch Filtration gewonnene Produkt wurde mit Wasser gewaschen und getrocknet.

### Beispiel 2: Katalysator A

1,11 g des Pd-Re-Salzes, hergestellt nach Beispiel 1, wurden bei 80°C in 20 g Wasser gelöst. 30 g eines Aktivkohleträger (Degussa 180 der Firma Degussa AG, Düsseldorf) wurden mit der Lösung des Pd-Re-Salzes bei 70°C getränkt. Sodann wurde der Katalysator bei 120°C in 100 NUh Stickstoff (N₂) getrocknet. Anschießend wurde 30 min bei gleicher Temperatur und 30 min. bei 200°C mit 0,5 % Wasserstoff (H₂) enthaltendem N₂ (100 NI/h) reduziert. Dann wurde für 1 h die Wasserstoffmenge auf 5 % und für weitere zwei Stunden auf 100 % erhöht. Danach wurde die Temperatur auf 400°C sowie der Fluss auf 3000 l/h H₂ erhöht. Die Heizraten betrugen dabei jeweils 5°C/min. Abschließend wurde der Katalysator nach Abkühlen in N₂ bei Raumtemperatur 7 h in 5 % Luft in N₂ passiviert. Der Katalysator enthielt 0,5 Gew.-% Pd und 2 Gew.-% Re.

### Beispiel 3

1,11 g des Pd-Re-Salzes wurden in 130 g Wasser bei 40°C gelöst. 10 g dieser Lösung wurden unter Rühren auf 30 g des Aktivkohleträgers (Degussa 180) aufgebracht. Sodann wurde der Katalysator 1 h bei 120°C getrocknet. Nach einem Waschschritt mit Wasser wurde die Tränk- und Trockenprozedur wiederholt bis die ganze Lösung auf den Träger aufgebracht war. Anschließend wurde der Katalysator, getrocknet und analog Beispiel 2 reduziert. Der Katalysator enthielt 0,5 Gew.-% Pd und 2 Gew.-% Re.

### Beispiel 4

20 g des Katalysators A wurden in einen Rohrreaktor eingefüllt und bei Atmosphärendruck und 150°C 2 h lang mit N₂ gespült (240 NI/h). Anschließend wurden 5 % H₂ zugemischt und nach zwei Stunden die Temperatur auf 200°C erhöht und über Nacht gehalten. Nach Umschalten auf eine 50 %ige H₂-N₂-Mischung wurde die Temperatur für 1 h auf 230°C erhöht und schließlich wurde eine weitere Stunde in 120 NI/h reinem H₂ reduziert. Abschließend wurde der Druck auf 40 bar erhöht. Mit diesem aktivierten Katalysator A wurde Bernsteinsäureanhydrid (BSA), das in einer Menge von 6.06 g/h als 20 gew.-%ige Lösung in gamma-Butyrolacton zudosiert wurde, bei 235°C und 40 bar in kontinuierlichen Betrieb hydriert. Das Molverhältnis H₂:BSA betrug 35. Es wurde bei einem Umsatz von 91 % eine Produktausbeute 81 % (39 % Tetrahydrofuran (THF) und 35 % γ-Butyrolacton) erzielt.

### Vergleichsbeispiel 1: Katalysator V1

60 g eines mit Wasser vorbefeuchteten Aktivkohleträgers (Degussa 180) wurde unter Rühren mit einer Lösung enthaltend 0,78 g Pd (NO₃)₂ 2H₂O und 1,52 g HReO₄ (72,8 gew.-%ige Lösung) in 20 ml Wasser bei Raumtemperatur getränkt. Anschlie-βend wurde der Katalysator analog der für Katalysator A in Beispiel 2 beschriebenen Trocknung und Reduktion behandelt. Der Katalysator enthielt 0,5 Gew.-% Palladium und 2 Gew.-% Rhenium.

### Vergleichsbeispiel 2:

20g des Katalysators V1 wurden analog Beispiel in einen Rohrreaktor eingebaut und analog Beispiel 5 aktiviert. Mit diesem aktivierten Katalysator A wurde Bernsteinsäureanhydrid (BSA), das in einer Menge von 5,94 g/h als 20 gew.-% Lösung in gamma-Butyrolacton zudosiert wurde, bei 235°C und 40 bar im kontinuierlichen Betrieb hydriert. Das Molverhältnis H₂:BSA betrug 35. Es wurde bei einem Umsatz von 80 % eine Produktausbeute von 77 % (9 % Tetrahydrofuran (THF) und 53 % γ-Butyrolacton).

## Patentansprüche

1. , Rheniumhaltiger Trägerkatalysator, **dadurch gekennzeichnet, dass** Rhenium und mindestens ein weiteres Metall der Gruppen VIII oder Ib des Periodensystems der Elemente, in Form mindestens eines Perrhenat-Doppelsalzes der allgemeinen Formel (I)
[Meₐ (NH₃)_{b} (OH)_{c}] (ReO₄)_{d}• eH₂O (I)
wobei Me für ein Metall der Gruppen VIII und Ib des Periodensystems der Elemente, insbesondere für Ru, Rh, Pd, Os, Ir, Pt, Cu, Ni, Ag oder Co steht, a 1 oder 2 bedeutet, b eine ganze Zahl von 1 bis 8, c eine ganze Zahl von 0 bis 5, d 2, 3 oder 4 und e eine ganze Zahl von 0 bis 12 darstellt, als bimetallische Vorläuferverbindung auf den Träger aufgebracht wird.

2. Rheniumhaltige Trägerkatalysatoren nach Anspruch 1, **dadurch gekennzeichnet, dass** als bimetallische Vorläuferverbindung Pd (NH₃)₄ (ReO₄)₂ und/oder Pt (NH₃)₄ (ReO₄)₂ eingesetzt wird.

3. Rheniumhaltige Trägerkatalysatoren, nach einem der Ansprüche 1 oder 2, **dadurch gekennzeichnet, dass** Rhenium und das weitere Metall der Gruppe VIII oder Ib des Periodensystems der Elemente in einer Menge von jeweils 0,03 bis 30 Gew.-%, bezogen auf den gesamten Katalysator, vorhanden sind.

4. Rheniumhaltige Trägerkatalysatoren, nach einem der Ansprüche 1 bis 3, **dadurch gekennzeichnet, dass** das elektronenmikroskopisch bestimmte Verhältnis von Rhenium zum Metall (Me) der Gruppe VIII oder Ib des Periodensystems der Elemente über den gesamten Katalysatorpartikel in 98 % der analysierten Punkte Schwankungen um einen Faktor kleiner 5 aufweist.

5. Verfahren zur Herstellung von Gemischen aus Tetrahydrofuran und gamma-Butyrolacton durch katalytische Hydrierung von Carbonylverbindungen, **dadurch gekennzeichnet, dass** ein Katalysator nach einem der Ansprüche 1 bis 4 verwendet wird.

6. Verfahren nach Anspruch 5, **dadurch gekennzeichnet, dass** die Carbonylverbindung ausgewählt ist aus Aldehyden, Carbonsäuren, Estern, Anhydriden und/oder Lactonen.

7. Verfahren nach Anspruch 6, **dadurch gekennzeichnet, dass** die Carbonylverbindung ausgewählt ist aus Maleinsäure, Fumarsäure, Bernsteinsäure oder Estern oder Anhydriden davon.

8. Verfahren nach einem der Ansprüche 5 bis 8, **dadurch gekennzeichnet, dass** die Hydrierung ist der Flüssigphase an fest angeordneten Katalysatoren bei einem Druck im Bereich von 5 bis 220 bar und einer Temperatur im Bereich von 110 bis 250°C durchgeführt wird.

## Claims

1. A supported rhenium catalyst, wherein rhenium and at least one further metal of groups VIII or Ib of the Periodic Table of the Elements, in the form of at least one perrhenate double salt of the general formula (I)
[Meₐ (NH₃)_{b} (OH)_{c}] (ReO₄)_{d} **•** eH₂O (I)
where Me is a metal of groups VIII and Ib of the Periodic Table of the Elements, in particular Ru, Rh, Pd, Os, Ir, Pt, Cu, Ni, Ag or Co, a is 1 or 2, b is an integer from 1 to 8, c is an integer from 0 to 5, d is 2, 3 or 4, and e is an integer from 0 to 12, is applied to the support as a bimetallic precursor compound.

2. The supported rhenium catalyst according to claim 1, wherein the bimetallic precursor compound used is Pd (NH₃)₄ (ReO₄) 2 and/or Pt (NH₃) ₄ (ReO₄)₂.

3. The supported rhenium catalyst according to either of claims 1 and 2, wherein rhenium and the further metal of group VIII or Ib of the Periodic Table of the Elements are present in an amount each of from 0.03 to 30% by weight, based on the entire catalyst.

4. The supported rhenium catalyst according to any of claims 1 to 3, wherein the ratio determined by electron microscopy of rhenium to metal (Me) of group VIII or Ib of the Periodic Table of the Elements over the entire catalyst particle shows deviations by a factor of less than 5 in 98% of the analyzed points.

5. The process for preparing mixtures of tetrahydrofuran and gamma-butyrolactone by catalytic hydrogenation of carbonyl compounds, which comprises using a catalyst according to any of claims 1 to 4.

6. The process according to claim 5, wherein the carbonyl compound is selected from aldehydes, carboxylic acids, esters, anhydrides and/or lactones.

7. The process according to claim 6, wherein the carbonyl compound is selected from maleic acid, fumaric acid, succinic acid or esters or anhydrides thereof.

8. The process according to any of claims 5 to 8, wherein the hydrogenation is carried out in the liquid phase over fixed bed catalysts at a pressure in the range from 5 to 220 bar and a temperature in the range from 110 to 250°C.

## Revendications

1. Catalyseur sur support contenant du rhénium, **caractérisé en ce que** du rhénium et au moins un autre métal du groupe VIII ou Ib du système périodique des éléments sont, sous la forme d'au moins un sel double de perrhénate de la formule générale (I) :
[Meₐ(NH₃)_{b} (OH)_{c}] (ReO₄) _{d}•eH₂O (I)
dans laquelle Me représente un métal des groupes VIII et Ib du système périodique des éléments, en particulier Ru, Rh, Pd, Os, Ir, Pt, Cu, Ni, Ag ou Co, a représente 1 ou 2, b est un nombre entier de 1 à 8, c un nombre entier de 0 à 5, d vaut 2, 3 ou 4, et e est un nombre entier de 0 à 12, appliqués sur le support comme composé précurseur bimétallique.

2. Catalyseur sur support contenant du rhénium selon la revendication 1, **caractérisé en ce que**, comme composé précurseur bimétallique, on met en oeuvre du Pd(NH₃)₄(ReO₄)₂ et/ou du Pt(NH₃)₄(ReO₄)₂.

3. Catalyseur sur support contenant du rhénium, suivant l'une des revendications 1 et 2, **caractérisé en ce que** le rhénium et l'autre métal du groupe VIII ou Ib du système périodique des éléments sont présents en une quantité de chacun 0,03 à 30 % en poids, par rapport au catalyseur total.

4. Catalyseur sur support contenant du rhénium, suivant l'une des revendications 1 à 3, **caractérisé en ce que** le rapport déterminé par microscopie électronique entre le rhénium et le métal (Me) du groupe VIII ou Ib du système périodique des éléments au sujet de la totalité des particules de catalyseur dans 98 % des points analysés présente des variations d'un facteur inférieur à 5.

5. Procédé de préparation de mélanges de tétrahydrofuranne et de gamma-butyrolactone par hydrogénation catalytique de composés carbonyle, **caractérisé en ce qu'**on utilise un catalyseur suivant l'une des revendications 1 à 4.

6. Procédé suivant la revendication 5, **caractérisé en ce que** le composé carbonyle est choisi parmi des aldéhydes, des acides carboxyliques, des esters, des anhydrides et/ou des lactones.

7. Procédé suivant la revendication 6, **caractérisé en ce que** le composé carbonyle est choisi parmi de l'acide maléique, de l'acide fumarique, de l'acide succinique ou leurs esters ou anhydrides.

8. Procédé suivant l'une des revendications 5 à 7, **caractérisé en ce que** l'hydrogénation est effectuée dans la phase liquide sur des catalyseurs agencés de manière fixe, à une pression de l'ordre de 5 à 220 bars et à une température de l'ordre de 110 à 250°C.
